# EUROPEAN PATENT APPLICATION

(11) **EP 3 620 134 A1**
(43) Date of publication of application: **11.03.2020**
(21) Application number: 19020513.8
(22) Date of filing: 05.09.2019
(51) Int. Cl.: A61F 2/24

(54) **SELF-EXPANDING MEDICAL DEVICE FOR SEALING PERIVALVULAR LEAKAGE**

(30) Priority: 06.09.2018 PL 42693518
(71) Applicant: Balton Sp. z o.o., 00-496 Warszawa (PL)
(72) Inventor: Plowiecki, Emil, 00-496 Warszawa (PL); Hurkala, Leszek, 00-496 Warszawa (PL)
(74) Representative: Orlinska, Dorota Irena

(57) **Abstract**

A self-expanding medical device is shown for paravalvular leak closure, comprising a flexible, braided structure composed of many metal strands, having in its free state a fixed, unfolded form with a developed shape, deformable to a form with a smaller cross-section than the unfolded form, wherein the device has a proximal end and a distal end and both ends include elements grouping and fixing the ends of the metal strands, **characterised in that** the unfolded form comprises three parts having a developed surface, a distal part **(1)**, a proximal part **(2)** and a middle part **(5)** between them with a cross-section smaller than the cross-sections of the distal **(1)** and proximal **(2)** parts, wherein the said three parts **(1, 2, 5)** are separated by narrowings of the braided structure, in the form of connecting elements **(3, 4)** with a cross-section smaller than the cross-section of these three parts **(1, 2, 5),** wherein each of the parts **(1,2,5)** is spatially shaped so that in each of them, in a free unfolded state, the distal wall and the corresponding proximal wall do not contact each other.

## Description

The object of the invention is a self-expanding medical device for paravalvular leak closure.

The invention relates to the field of intravascular medical devices introduced by percutaneous procedure into the patient's body in order to carry out medical procedures. The device is made of metal (metal alloys) with shape memory and is formed of a flexible, braided structure composed of many metal strands (metal threads, wires) and in a free state has a fixed, unfolded form which is deformable to a form with a smaller cross-section than the unfolded form. The device at its proximal and distal ends has elements grouping and fixing together the ends of the metal strands, which prevents these strands from tangling and keeps the entire structure of the device in a predetermined shape.

The device is therefore of a low profile, i.e. it is possible to maintain its folded position, close to the tubular position, having a small diameter, in order to place the device in an intravascular catheter and introduce it through such a catheter to the destination in the patient's body. The device belongs to the group of devices for the treatment of congenital and acquired heart defects, the device being intended for the treatment of paravalvular leak (PVL). Paravalvular leaks are one of the complications observed in patients after implantation of a cardiac prosthetic valve. Frequency of their occurrence is estimated to be 2-3%. Leakage can be caused by tissue damage in the vicinity of the valve, paravalvular infection, interruption of suture continuity or thrombus formation. The method of PVL treatment is a cardiac surgery, but for several years the alternative has been the non-invasive percutaneous procedure of closure of PVL. Detailed descriptions of treating patients with this method are widely known from the literature, for example from the publication: Iwona Świ 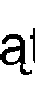 tkiewicz et al. Percutaneous closure of mitral perivalvular leak (Przezskórne zamkni 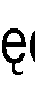 cie mitralnego przecieku okotozastawkowego), *Kardiol Pol* 2009; 67: 762-764.

The use of the percutaneous procedure, despite the undoubted benefits associated with avoiding cardiac surgery, is also associated with possible complications, and the procedure itself is complicated and requires a lot of experience. Some of the adverse side effects after percutaneous procedure may be related to the type of closure device, its shape, structure and method of implantation.

Therefore, there is still a need to develop new closure devices adapted to the anatomical conditions at the implantation site. Parameters such as the shape of the closure device, its rigidity and the actual ability to block (closure, plug) leakage are particularly important. It should be emphasised that devices of this type must reliably fulfil their role on a working heart, i.e. with variable pressures and cyclical deformations.

Closure devices based on the technology of weaving a spatial mesh structure of wires made of metal with shape memory are known in the art. Devices of this type are assembled, i.e. when they are placed at the implantation site, e.g. in the patient's heart, they have a self-settling form shaped in a predetermined way (spatially developed form), which is achieved by means of the metal with shape memory used. This form has transverse dimensions, i.e. cross-sectional areas larger than the folded form, placed in an intravascular catheter and prepared for introduction into the patient's body. The device is flexible and when placed in the catheter is longitudinally stretched in order to minimise its diameter, i.e. more generally its cross-sectional area. In this form, the device is subjected to a force limiting it in the catheter. In other words, this form of the device is immobilised in the catheter, and the target form to which the device automatically strives in its free state, the form with the developed shape, is the form released from the catheter. The above principles of operation of this type of devices are known in the art and are also used in the present description of the invention.

From international patent application WO9742878A1, a foldable intravascular medical device is known, comprising a woven metal tubular structure having a proximal end and a distal end, wherein each end is closed with bonding elements, wherein this woven metal structure has a predetermined expanded configuration shaped to close the abnormal opening in the body organs, and said expanded configuration is deformable to a smaller cross section for introducing by the catheter, wherein the metal fabric exhibits the property of shape memory, so that the medical device tends to return to said predetermined extended configuration when it is not limited, wherein the extended configuration comprises two parts (proximal and distal) having an extended diameter and a part having a reduced diameter sandwiched between the two parts having an extended diameter.

Other types of self-expanding devices for closing defects or deformations in the heart, constructed of a woven structure formed of many metal strands joined together in fastening sleeves are disclosed, for example, in publications WO1999012478A1, WO2005020822A1, WO2010030485A1.

The object of the invention is a self-expanding medical device for paravalvular leaks closure, comprising a flexible, braided structure composed of many metal strands, having in its free state a fixed, unfolded form with a developed shape, deformable to a form with a smaller cross-section than the unfolded form, wherein the device has a proximal end and a distal end and both ends include elements grouping and fixing the ends of the metal strands, characterised in that the unfolded form comprises three parts having a developed surface, a distal part, a proximal part and a middle part between them with a cross-section smaller than the cross-sections of the distal and proximal parts, wherein the said three parts are separated by narrowings of the braided structure, in the form of connecting elements, with a cross-section smaller than the cross-section of these three parts, wherein each of the parts is spatially shaped so that in each of them, in a free unfolded state, the distal wall and the corresponding proximal wall do not contact each other.

Preferably, the middle part of the device comprises a sealing material.

Preferably, the distal part of the device comprises a stiffening means.

Preferably, the cross section of the middle part of the device has the form of a square, a rectangle, a circle, an ellipse, an hourglass.

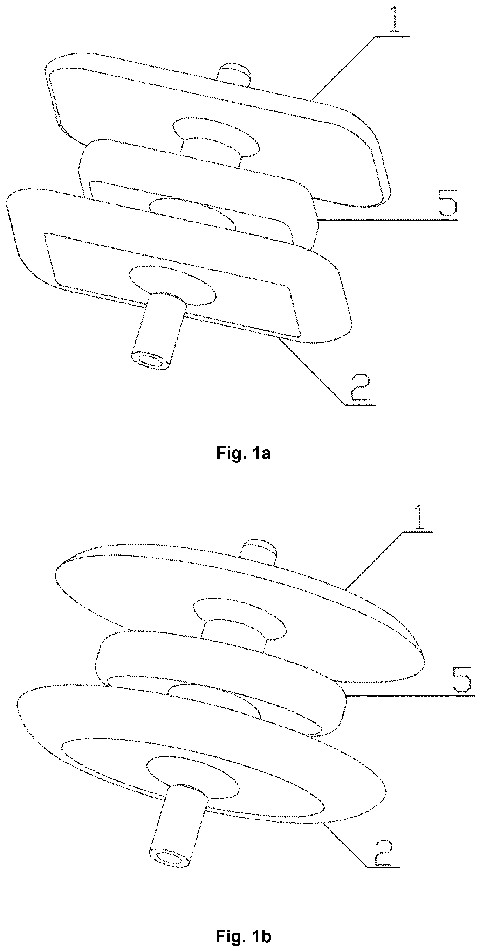

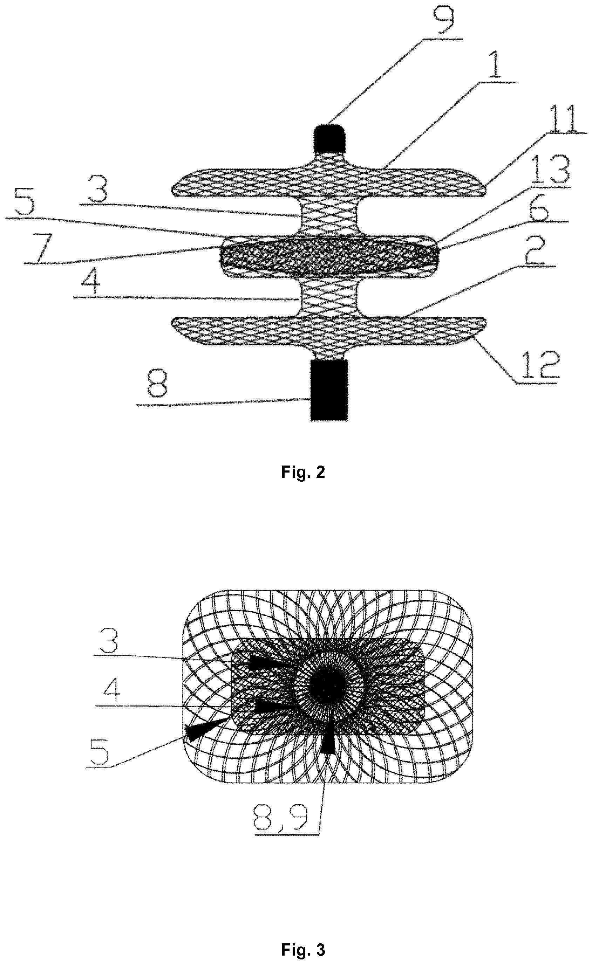

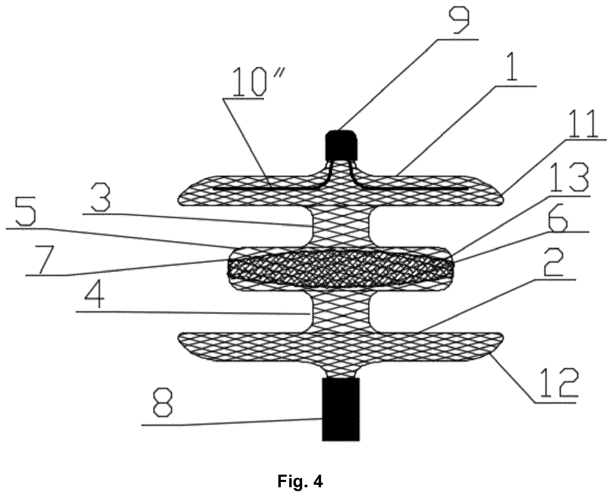

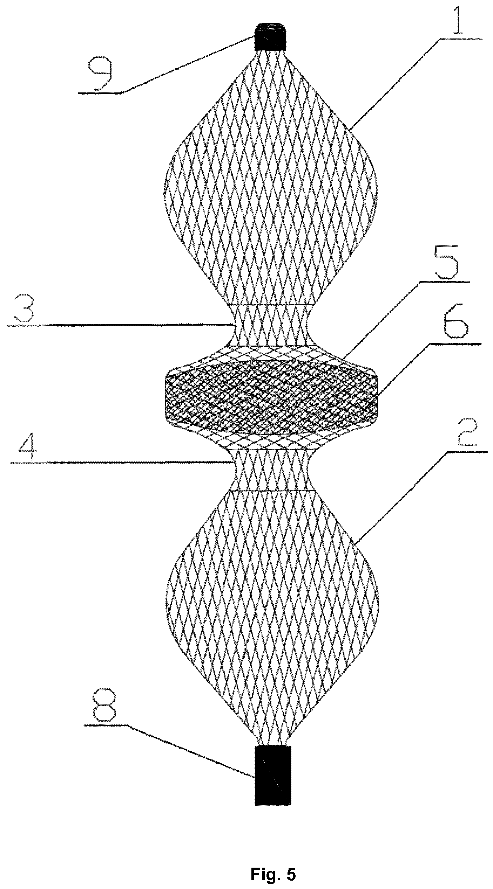

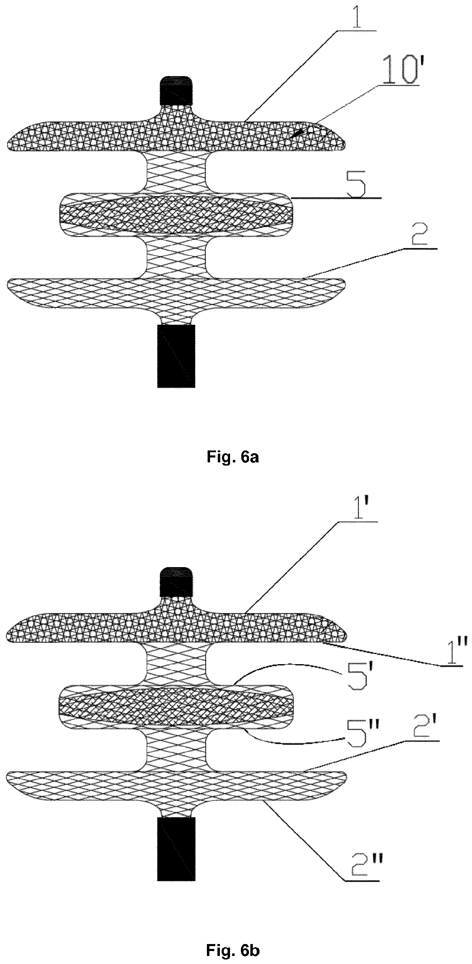

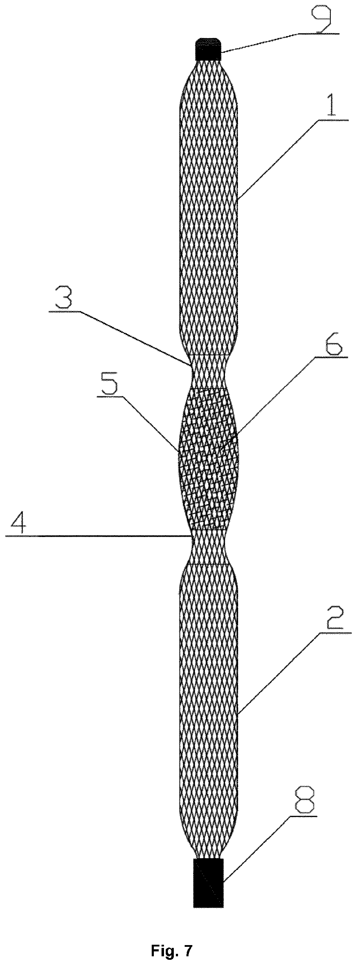

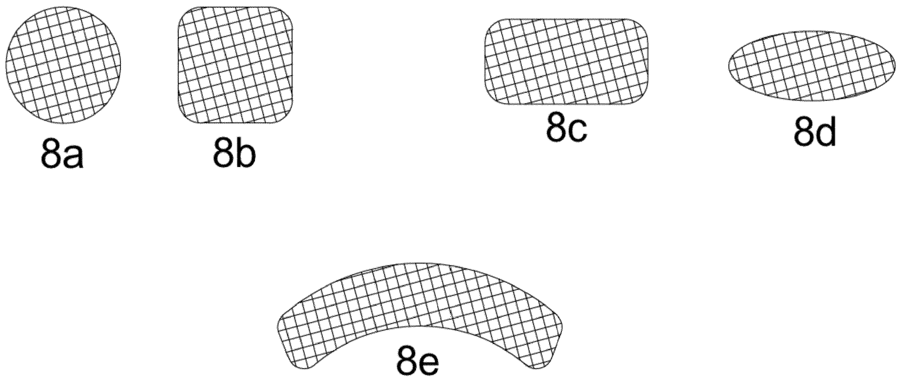

The object of the invention in an exemplary embodiment is shown in the drawing in which Fig. 1a shows a general outline of the shape of the device in an unfolded form (in a free state), in which three parts with a developed surface have a rectangular cross-section, Fig. 1b shows a similar overall view of the device in which three parts with a developed surface have an ellipsoidal cross-section, wherein Fig. 1a and 1b do not show the structure of the device woven from metal strands, but only, in simplification, the achieved general shape of the device, Fig 2 shows the device from Fig. 1a or 1b in a side view, with the structure of woven metal strands made visible, wherein in the middle part, the sealing material fixed with threads to the metal strands is made visible, Fig. 3 shows the device from Fig. 1a in a view from the side of a distal mounting sleeve, wherein due to the poor visibility of overlapping metal strands, relevant parts in the drawing are shown by arrows, Fig. 4 shows additional stiffening in the form of loops in the distal part of the device, Fig. 5 shows the device in partially, longitudinally stretched form with a sealing material in its middle part, Fig. 6a and 6b show the device in a side view in a free (unfolded) state with additional stiffening embedded in the distal part from the side of the distal tip, in the form of additional metal strands woven in the basic structure of the device, wherein Fig 6b shows additionally marked distal and proximal walls of the three parts of the device, Fig. 7 shows a fully folded (stretched out) device prepared for placement in an intravascular catheter, and Figs. 8a, 8b, 8c, 8d and 8e show various exemplary cross-sectional shapes of the centre part of the device.

The self-expanding medical device according to the invention is characterised in that its unfolded form in a free state comprises three parts spatially shaped in a predetermined manner with an expanded surface, a distal part **1,** a proximal part **2** and placed between them a middle part **5,** having a cross section smaller than the cross sections of the distal part **1** and the proximal part **2.** The said three parts **1, 2, 5** of the device are formed from an originally tubular structure of woven metal strands, wherein this structure is then shaped by means of appropriate treatment into the form of three parts successively passing into each other, which have a contour of flattened (towards the longitudinal axis) solids (bodies). Starting from its distal side, the device consists successively of a distal sleeve **9** mounting metal strands, a distal part **1** with an contour of a closed flattened body having a distal wall **1',** a proximal wall **1"** preferably parallel to it, distant by a certain distance, and a side wall **11** connecting them, behind the distal part **1** there is a first connecting element **3** in the form of a narrowing of the woven structure, followed by the middle part **5** with the contour of a closed flattened body having a distal wall **5'** and a proximal wall **5"** preferably parallel to it, placed at a distance, and a side wall **13** connecting them, then, behind the middle part **5** there is a second connecting element **4** in the form of a narrowing, followed by the proximal part **2** with the contour of a closed flattened body having a distal wall **2',** a proximal wall **2"** preferably parallel to it, distant by a certain distance, and a side wall **12** connecting them, and then the device is narrowed and at the proximal end there is a proximal sleeve **8** fixing the ends of the metal strands, wherein the proximal sleeve **8** is preferably elongated in relation to the distal mounting sleeve **9** and is preferably provided internally, from the proximal side, with a threaded hole, ensuring fixing of the device to further elements of an intravascular, percutaneous introducing system comprising a suitably matched, externally threaded element.

This type of joint is known to those skilled in the art and is not the object of the invention, and therefore the proximal sleeve may also have other external and internal shapes, wherein it is preferably used not only to connect the ends of metal strands, but also to fix the device in the system introducing and implanting the device in the target position in the patient's body, and also allows the device to be removed by means of a catheter.

In a preferred embodiment, the three parts of the device **1, 2, 5** have distal walls **1', 5', 2'** substantially parallel to each other and corresponding proximal walls **1", 5", 2",** preferably the device has all six walls parallel to each other in the unfolded (free) state. "Substantially" means that most of the surface of said walls are parallel to each other, because in the vicinity of connecting elements (narrowings) and at the ends of the three parts having an expanded surface there are portions smoothly passing into each other with rounded edges.

These edges, i.e. the side walls **11** and **12** of the extreme parts of the device, i.e. of the distal part **1** and the proximal part **2,** have a different shape than the edge, i.e. the side wall **13,** of the middle part **5,** because the side walls **11** and **12** from the distal and proximal sides are obliquely rounded, while the side wall **13** of the middle part **5** is substantially perpendicular to the distal **5'** and proximal **5"** walls of the middle part **5** (and wall **13** is parallel to the axis of the device). The side walls **11, 12, 13** of the three parts **1, 2, 5** ensure that the distance between the distal walls **1', 5', 2'** and the corresponding proximal walls **1", 5", 2"** is maintained, wherein the perpendicular side wall **13** maintains higher stiffness (lower susceptibility to deformation) of the middle part **5** compared to the oblique side walls of the distal **1** and proximal **2** parts. Therefore, the obliquely rounded side walls **11** and **12** allow for a greater degree of possible deformation, adaptation of the distal and proximal parts of the device (and thus the location of the entire device) to the morphology in the area of the leak, or adaptation to the vicinity of the previously implanted valve.

The distal part of the device is, from the side of the distal fixing sleeve, preferably on its distal wall, optionally provided with stiffening means **10', 10",** which may have the form of additional wires of various shapes, e.g. S-shaped or spiral, the shape of the stiffening loop **10'** (as shown in side view in Fig. 4) or constitute many additional wires woven into the base structure of the device and create a braided stiffening structure **10".** The stiffening elements are fixed in the fixing sleeve together with the metal strands of the base structure. Such stiffening elements change the characteristics of the device and allow the operator to anchor the device more precisely. The distal part is then less prone to stretching out compared to the other parts, which makes transcatheter positioning to be more easily and more efficiently performed by the operator. The risk of moving or falling out of the device during the implantation procedure is reduced.

The middle part **5** of the device is provided with a sealing material **6** fixed to the walls of this part **5** by means of fixing means e.g. threads **7.**

For the purposes of the present invention, the construction of the device is discussed with the assumption that its longitudinal axis extends through the fixing sleeves. Thus, when the device is stretched out as shown in Fig. 7, it is stretched along its longitudinal axis. When describing the length of the device, it is the size calculated along the said axis. In accordance with this convention, the proximal and distal ends of the device are defined. Consequently, when the width of the device is indicated, it refers to the sizes of the cross-sections of the device in a plane perpendicular to said longitudinal axis.

Accordingly, the exemplary shapes shown in Fig. 8 relate to such cross-sections of the middle part of the device.

According to this convention, when discussing the expanded shape of the device in the free state and the developed surfaces of its individual parts, it refers to the free state of the device when its length in the longitudinal axis is shortened and its transverse dimensions increase, i.e. the width of the three parts with developed surface increases.

Similarly, when, for the purposes of the present invention, the condition is made that, in the unfolded state, the proximal and distal walls of the three parts of the device do not touch each other, this refers to the creation of a distance, a free space, between these walls in the longitudinal axis of the device.

In a preferred embodiment of the invention, the extreme parts of the device, i.e. the proximal and distal parts, in the longitudinal axis, have a smaller size (thickness) than in the transverse direction (at least one of them), i.e. the said distance between the proximal and distal walls in each of the two extreme parts, the proximal and distal parts, of the device is smaller than the transverse dimensions of these parts. The proximal and distal parts may have an elongated shape in cross section e.g. the shape of an elongated rectangle. At least one of the sides of the rectangle is much larger than the distance between the proximal and distal walls of these parts.

The device according to the invention has a middle part with a developed surface, with smaller transverse dimensions than the extreme, distal and proximal, parts. Fig. 5 shows how the parts of the device behave when it begins to stretch out. The middle part is not significantly deformed in terms of cross-section, which is desirable because it is the main closure element of the device. In a situation where the plugged leakage hole is relatively long, the outer (distal and proximal) parts are deformed, while the middle part has a stabilising function.

In one embodiment, the device according to the invention may have different cross-sectional shapes of all three parts with a developed surface. It is preferred that the three parts have an analogous cross-sectional shape, e.g. rectangular or elliptical, as shown in Figs. 1a and 1b, but the middle part has a smaller cross-sectional area.

Figs. 8a, 8b, 8c, 8d, 8e show various exemplary cross-sectional shapes of the middle part of the device, which may have perfectly symmetrical forms and be circular or square (Figs. 8a and 8b), or elongated forms (with a lower degree of symmetry) of rectangular or elliptical shape (8c and 8d) or arcuate forms (8e). Shown cross-sectional shapes of the middle part of the device can also take other, arbitrary shapes adapted to the morphology of the paravalvular leak and may have e.g. the shape of a pear, an hourglass, a heart, a triangle, a rhomboid, etc.

Similarly, the cross-sections of the distal and proximal parts of the device may have a variety of shapes as shown above.

In relation to the concept of sealing material, it is known from the prior art. It can be a woven fabric, a polymer nonwoven fabric or it can be threads of a fabric directly woven on the warp of the base structure of metal strands. The sealing material can be fixed, sewn with threads. It is also possible to fix the closure fabric in a different way, e.g. by weaving its fibers directly into the metal strands.

Optionally, in the device according to the invention also the distal and proximal parts can have an embedded sealing material. It can be applied only on one of the walls of these parts, i.e. either on the distal wall or on the proximal wall, or it can be placed on the entire distal or proximal part or on both parts. It is also possible to completely cover the device with a sealing fabric.

It will be obvious to a person skilled in the art that the device has all of its edges rounded so that they do not cause irritation to the patient's tissues. Therefore, for example, when it is indicated that the cross-sections of parts of the device are square or rectangular, it is known that the corners of these elements are rounded, and thus they are only approximately square/rectangular.

The device structure described above ensures more effective closure of the paravalvular leak and is well adapted to the varied morphology of leaks. The middle part of the device has a smaller cross-sectional area than the other extreme parts of the device and is therefore less susceptible to deformation. Furthermore, closure agents added in the middle part more effectively block the leakage. The proximal and distal parts have a varied shape, can be elongated (in terms of cross-sectional area) and can be positioned precisely relative to the implanted valve. This allows more effectively blocking the leak but also prevents the formation of another, secondary leak. The benefits of placing the walls of the three parts of the device at a distance from each other, while maintaining the space between the proximal and distal walls in each of these three parts, are also important. On the one hand, the spatial form of the device is more durable, and on the other hand, the leakage is blocked by a multiplied number of walls that can slide slightly relative to each other, which, in multiple layers, exerts closure and clamping forces in slightly different directions. Separating the proximal and distal walls from each other in the three parts of the device also allows placing the closure material separately on each wall, which will multiply the operation of the device.

### Reference signs in the drawings:

1 distal part
1' distal wall of the distal part
1" proximal wall of the distal part
2 proximal part
2' distal wall of the proximal part
2" proximal wall of the proximal part
3 first connecting element (connecting the distal part with the middle part)
4 second connecting element (connecting the middle part with the proximal part)
5 middle part
5' distal wall of the middle part
5" proximal wall of the middle part
6 sealing material
7 threads
8 proximal fixing sleeve
9 distal fixing sleeve
10' stiffening means in the form of a braided structure
10"stiffening means in the form of a loop
11 side wall of the distal part
12 side wall of the proximal part
13 side wall of the middle part

## Claims

1. A self-expanding medical device for paravalvular leak closure, comprising a flexible, braided structure composed of many metal strands, the device has in its free state a predetermined, unfolded form with a expanded shape and is deformable to a form with a smaller cross-section than the unfolded form, wherein the device has a proximal end and a distal end and both ends include elements grouping and fixing the ends of the metal strands, **characterised in that** the unfolded form comprises three parts having a developed surface, a distal part (**1**), a proximal part (**2**) and placed between them a middle part (**5**) having a cross-section smaller than the cross-sections of the distal (**1**) and proximal (**2**) parts, wherein the said three parts (**1, 2, 5**) are separated by narrowings of the braided structure, in the form of connecting elements (**3, 4**) with a cross-section smaller than the cross-section of these three parts (**1, 2, 5**), wherein each of the parts (**1, 2, 5**) is spatially shaped so that in each of them, in a free unfolded state, the distal wall and the corresponding proximal wall do not contact each other.

2. The medical device according to claim 1, **characterised in that** its middle part (**5**) comprises a sealing material.

3. The medical device according to claim 1 or 2, **characterised in that** its distal part (**1**) comprises a stiffening means.

4. The medical device according to claim 1 or 2, or 3, **characterised in that** the cross section of the middle part (**5**) has the form of a square, a rectangle, a circle, an ellipse, an hourglass.
